# EUROPÄISCHE PATENTSCHRIFT

(11) **EP 0 821 929 B1**
(45) Veröffentlichungstag und Bekanntmachung des Hinweises auf die Patenterteilung: **15.09.2004**
(21) Anmeldenummer: 97112804.6
(22) Anmeldetag: 25.07.1997
(51) Int. Cl.: A61H 1/00, A61B 5/103, A61B 7/00

(54) **Trainingsgerät mit einer Vorrichtung zur Durchführung eines Verfahrens zum Stimulieren des Knochenwachstums**
Training device for implementing a method for stimulation of bone tissue growth
Dispositif pour réaliser un procédé de stimulation destiné aux tissus osseux

(30) Priorität: 03.08.1996 DE 19631493
(43) Veröffentlichungstag der Anmeldung: 04.02.1998
(73) Patentinhaber: Schwalbe, Hans-Joachim, Prof. Dr., 35753 Greifenstein-Holzhausen (DE); Franke, Ralf-Peter, Prof. Dr. Dr., 89160 Dornstadt/Temmenhausen (DE); Dörner, Peter, Dr. Dr., 94081 Fürstenzell (DE)
(72) Erfinder: Schwalbe, Hans-Joachim, Prof. Dr., 35753 Greifenstein-Holzhausen (DE); Franke, Ralf-Peter, Prof. Dr. Dr., 89160 Dornstadt/Temmenhausen (DE); Dörner, Peter, Dr. Dr., 94081 Fürstenzell (DE)
(74) Vertreter: Hebing, Norbert

(56) Entgegenhaltungen:
- WO-A-93/24092
- DE-A- 1 918 716
- DE-A- 4 422 451

## Beschreibung

Die Erfindung betrifft eine Vorrichtung zur Aufnahme und Auswertung von während der Belastung in menschlichen Gelenken und/oder Knochen auftretenden Geräuschen, welche einen ungedämpften, resonanten, piezoelektrischen Wandler als Schallaufnehmer hat, der auf der Hautoberfläche eines Menschen aufsetzbar ist, und die eine Auswertelektronik aufweist, welche die von dem Schallaufnehmer während der Belastung aufgenommenen Geräusche hinsichtlich des Auftretens von Mikrorissen im Knochen analysiert und daraus ein Warnsignal ableitet, falls die Belastung so hoch ist, dass eine Schädigung von Gelenken und/oder Knochen zu befürchten ist. Weiterhin betrifft die Erfindung ein Trainingsgerät mit einer solchen Vorrichtung.

Es ist in der Medizin allgemein bekannt, dass das Knochenwachstum durch Belastung wesentlich gesteigert werden kann. Deshalb sollten Menschen nach Knochenbrüchen möglichst früh das betreffende Gliedmaß trainieren. Ebenso wachsen Implantate rascher in die Knochensubstanz ein, wenn eine hohe Vorspannung zwischen dem Implantat und dem Knochen erzeugt wird. Das Maß der Belastung beim Trainieren wird in der Praxis aufgrund von Erfahrungen festgelegt. Da eine zu hohe Belastung zu einem erneuten Brechen des Knochens und bei Implantaten eine zu hohe Vorspannung zu einer Schädigung des angrenzenden Knochens führen kann, ist es derzeit erforderlich, eine wesentlich niedrigere Belastung oder Vorspannung zu wählen, als es für die Heilungsgeschwindigkeit optimal wäre. Man nimmt deshalb aus Sicherheitsgründen einen längeren Heilungsprozess in Kauf.

Die DE-A-44 22 451 A1 beschreibt bereits ein orthopädisches Untersuchungsverfahren, bei dem ein Schallaufnehmer auf die Hautoberfläche in dem zu überwachenden Körpersegment aufgesetzt und mittels einer Auswertelektronik die Schallsignale des Schallaufnehmers analysiert werden, welche während der Belastung des Körpersegments des zu untersuchenden Menschen auftreten. Hierdurch kann man in-vivo die individuelle Bruchlast mechanisch belasteter menschlicher Knochen ermitteln. Weiterhin eignet sich das Untersuchungsverfahren gemäß dieser Schrift dazu, die Beschaffenheit von Gelenken und Knochenveränderungen zu erkennen. Bei dem Schallaufnehmer gemäß der DE-A-44 22 451 A1 handelt es sich um einen ungedämpften, resonanten, piezoelektrischen Wandler.

Durch die WO 93/24092 ist es auch schon bekannt, das Knochenwachstum zu stimulieren, indem die zu behandelnde Person sich auf eine Vorrichtung stellt oder setzt, welche Aktuatoren aufweist, die mit relativ hoher Frequenz schwingen und dabei relativ geringe Kräfte auf die Knochen ausüben. Es handelt sich bei dieser Vorrichtung nicht um ein Trainingsgerät, weil der Benutzer nicht aktiv trainiert, sondern passiv den Wirkungen von in ihn eingeleiteten Schwingungen ausgesetzt wird.

Der Erfindung liegt das Problem zugrunde, eine neue Verwendung für eine Vorrichtung der eingangs genannten Art herauszufinden. Weiterhin soll ein Trainingsgerät mit einer solchen Vorrichtung geschaffen werden.

Das erstgenannte Problem wird erfindungsgemäß durch die Verwendung der Vorrichtung in einem zur Stimulation des Knochenwachstums des Menschen ausgebildeten Trainingsgerät für gewerbliche Fitness-Studios oder den privaten Bereich zur Vermeidung einer Überbelastung von Knochen und/oder Gelenken während des Trainierens gelöst.

Diese erfindungsgemäße Verwendung beruht auf der Erkenntnis, dass es einen belastungsabhängigen Umbau von Knochen gibt, der bei gesunden Menschen zu einer raschen Verfestigung der Knochen führt. Dabei können die Knochen so weit belastet werden, dass sich Mikrorisse bilden, dass also eine unbedenkliche Rissbildungsgrenze gerade überschritten wird. Solchen Belastungen versuchte man bisher fernzubleiben, weil die Grenze zu einer makroskopischen Knochenschädigung, die jenseits der Mikrorissgrenze liegt, bislang nicht ausgelotet werden konnte. Erst durch die erfindungsgemäße Überwachung mittels eines Schallaüfnehmers in einem Trainingsgerät während der Steigerung der Belastung wird es möglich, beim Training diese Rissbildungsgrenze geringfügig, jedoch nicht wesentlich zu überschreiten. Durch die erfindungsgemäße Verwendung der Vorrichtung lässt sich nach operativen Eingriffen durch Training sehr rasch wieder eine hohe Festigkeit des betroffenen Knochens erreichen. Weiterhin bietet die erfindungsgemäße Verwendung Sportlern die Möglichkeit, durch gezieltes Training rasch eine höhere Festigkeit ihrer Knochen zu erreichen.

Das zweitgenannte Problem, nämlich die Schaffung eines Trainingsgerätes mit der vorgenannten Vorrichtung, wird erfindungsgemäß dadurch gelöst, dass die Auswertelektronik zusätzlich zur Auswertung der auf eine Rissbildung hinweisenden Geräusche zum Analysieren von auf eine Knorpelschädigung hinweisenden Geräuschen ausgebildet und das Trainingsgerät zur Anzeige dieser Geräusche ausgebildet ist. Ein solches Trainingsgerät ist insbesondere dann vorteilhaft, wenn die Gefahr besteht, dass bei höherer Belastung zunächst nicht die Knochen, sondern die Gelenke geschädigt werden.

Relativbewegungen zwischen dem Schallaufnehmer und der Haut, welche zu Störgeräuschen führen können, lassen sich dadurch vermeiden, dass der Schallaufnehmer mittels eines flüssigen, schallleitenden Klebers auf der Hautoberfläche befestigt wird. Diese Befestigungsart ist für das erfindungsgemäße Trainingsgerät sehr wichtig, weil es während der Bewegung des Menschen unter Belastung durchgeführt werden soll.

Der Schallaufnehmer des erfindungsgemäßen Trainingsgerätes liefert ein besonders eindeutiges Nutzsignal, wenn er innerhalb eines hutförmigen Gehäuses eine Sonde mit einem Piezoelement hat, wenn die Sonde durch eine elastische, schalldämpfende Zwischenschicht mit dem Gehäuse verbunden ist und wenn zwischen der inneren Rückseite des Gehäuses und der Sonde eine die Sonde zur offenen Stirnseite des hutförmigen Gehäuses vorspannende Druckfeder angeordnet ist. Durch diesen zweischaligen Aufbau mit Dämpfung in dem Zwischenbereich des äußeren Gehäuses und der Sonde ist der Schallaufnehmer unempfindlich gegenüber Reibbewegungen zwischen der Sonde und dem Befestigungssystem des Schallaufnehmers. Relativverschiebungen zwischen Haut und Piezoelement werden durch Membranspannungen der Haut, die durch das auf die vorgespannte Haut aufgeklebte Sondengehäuse erzeugt werden, vermieden. Er eignet sich auch dazu, den Zustand von Gelenken und Schäden im Stütz- und Bindegewebe bei der Festlegung der Belastung beim Training zu berücksichtigen.

Eine vorteilhafte Weiterbildung des Trainingsgerätes zeichnet sich dadurch aus, dass seine Lager schallabsorbierend in einem Stützring aus austenitischem Werkstoff oder aus einem Mehrschichtverbundwerkstoff eingebaut sind. Durch diese Gestaltung arbeitet das Trainingsgerät sehr geräuscharm, so dass es keine bei der Auswertung der Schallsignale störenden Geräusche erzeugt. Mit Hilfe einer solchen Vorrichtung wird der Mensch entweder passiv zwangsweise geführt bewegt oder aber er bewegt sich aktiv gegen eine definierte äußere Belastung. Durch die erfindungsgemäße Lagerung kommt es zu einer Dämpfung bzw. zu Vielfachreflexionen der Lagergeräusche, wodurch diese in ihrer Amplitude so gesenkt werden, dass sie unterhalb der Registrierschwelle des Messsystems liegen.

Auch Bewegungen des Menschen relativ zu der Vorrichtung führen nicht zu störenden Geräuschen, wenn die Vorrichtung Liege- oder Sitzflächen mit einer Auflage aus ungepolstertem, gummiertem Verbundwerkstoff aufweist.

Die Erfindung lässt verschiedene Ausführungsformen zu. Zu ihrer weiteren Verdeutlichung wird nachfolgend auf die Zeichnung Bezug genommen. Diese zeigt in
- Fig.1: eine Prinzipskizze eines an einem menschlichen Bein angebrachten Schallaufnehmers mit Messwerterfassung,
- Fig.2: eine Schnittdarstellung des Schallaufnehmers,
- Fig.3 - 7: Diagramme, welche die Schallemission über die Zeit bei verschiedenen Gegebenheiten dar stellen.

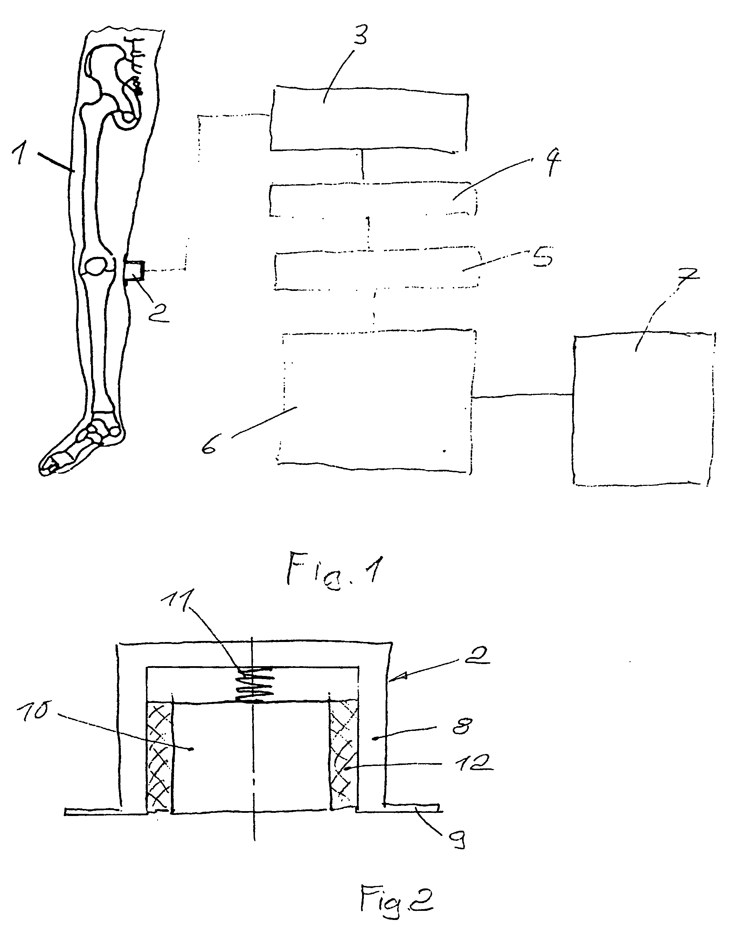

Die Figur 1 zeigt ein menschliches Bein 1, an welchem ein Schallaufnehmer 2 angebracht ist. Das Signal dieses Schallaufnehmers 2 wird einem Vorverstärker 3 mit Impedanzanpassung zugeführt. Von dort gelangt es über einen Bandpassfilter 4 und einen Zwischenverstärker 5 zu einer Messwerterfassung 6. Dort werden die folgenden Größen erfasst: RMS, I(t), U(t), F(t), F(t), F(t), V (t), V(t), V(t). Die ermittelten Werte werden einem Diagnosesystem mit einem Display 7 zugeführt und angezeigt. Dort erscheint auch ein Warnsignal, sobald die Belastung des Beines 1 so hoch wird, dass es zu einer Schädigung der Gelenke oder Knochen kommen kann.

Bei der Benutzung eines erfindungsgemäßen Trainingsgerätes wird während des Trainierens des Beines 1 die Belastung so lange gesteigert, bis die Rissbildungsgrenze geringfügig überschritten ist. Dadurch wird ein möglichst rasches Knochenwachstum stimuliert, ohne dass die Gefahr einer Schädigung besteht.

Die Figur 2 lässt den Aufbau des Schallaufnehmers 2 erkennen. Dieser hat ein hutförmiges Gehäuse 8 mit einem flanschartigen Rand 9. Mit diesem Rand 9 wird der Schallaufnehmer 2 mittels eines schallleitenden Klebers auf der Hautoberfläche befestigt. In dem Gehäuse 8 ist eine Sonde 10 angeordnet, welche ein nicht dargestelltes Piezoelement enthält. Eine Druckfeder 11, welche zwischen der rückwärtigen Stirnfläche der Sonde 10 und der inneren Rückwand des Gehäuses 2 gespannt ist, sorgt für eine definierte Anpresskraft der Sonde 10 auf die Hautoberfläche. Zwischen der Sonde 10 und der Haut befindet sich eine niederviskoses Gel oder ebenfalls ein hautverträglicher, gut schallleitender Kleber. Eine elastische Zwischenschicht 12 verbindet die äußere Mantelfläche der Sonde 10 mit dem Gehäuse 8, erlaubt jedoch die angestrebte axiale Vorspannung.

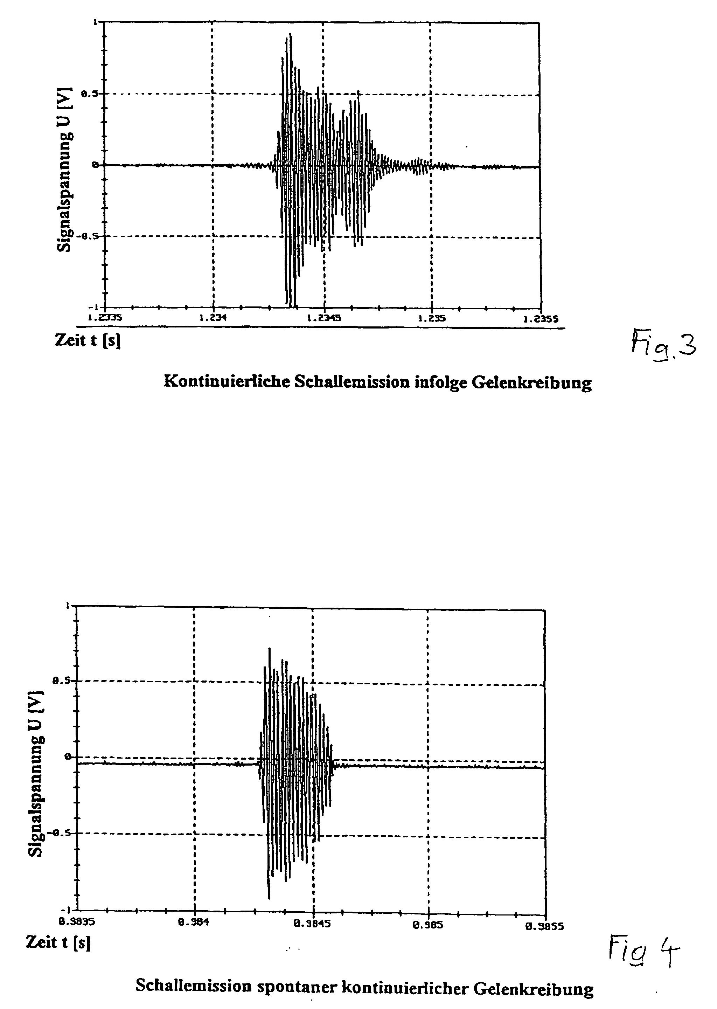

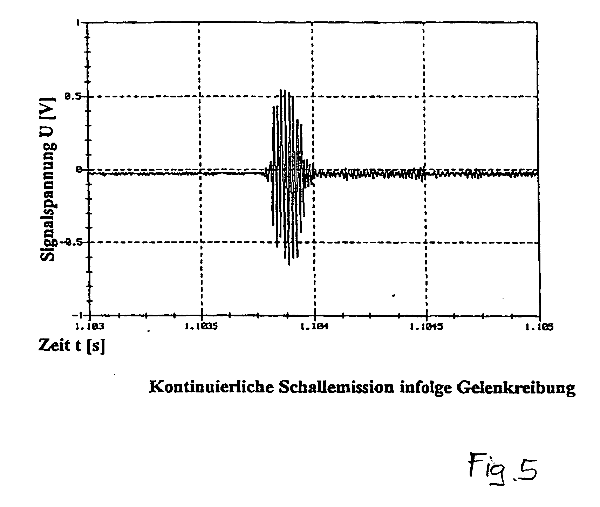

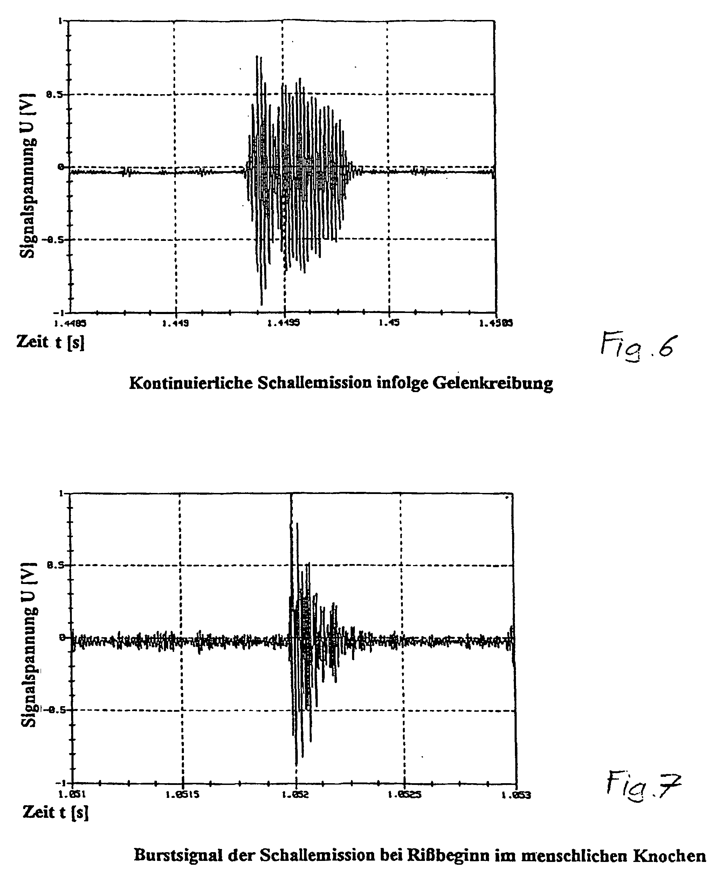

In Figur 3 ist die kontinuierliche Schallemission infolge Gelenkreibung, in Figur 4 die Schallemission spontaner, kontinuierlicher Gelenkreibung gezeigt. Die Figuren 5 und 6 geben weitere Beispiele für die kontinuierliche Schallemission infolge Gelenkreibung, während die Figur 7 die Schallsignale bei Rissbildungsbeginn im menschlichen Knochen zeigt. Alle Diagramme wurden während der Bewegung eines Gelenkes unter Last aufgezeichnet. Zu erkennen ist, dass die Signale der Schallemission, die von Stick-Slip-Effekten, Oberflächenrauhigkeiten und massiven Verschleißerscheinungen bzw. entzündlichen Veränderungen am Gelenk bei Bewegung verursacht werden, sich prinzipiell im zeitlichen Verlauf der Signalamplitude unterscheiden. Reibgeräusche zeigen den typischen Signalverlauf mit kontinuierlicher Emission. Reibung zwischen Gelenkknorpel und Gegenlager sowie viskoelastische Verformung des Gelenkknorpels unter spontaner Lasteinleitung verursachen Schallemissionssignale mit einem allmählichen Einschwingen auf Maximalamplitude. Verschleißschäden sind bei der Bewegung des menschlichen Gelenkes unter Last gekennzeichnet durch hohe Amplituden und hohe Schallenergien.

Wenn auf den Beginn einer Rissbildung geschlossen werden soll, wird aus der Winkellage des Gelenkes, der mechanischen Belastung und des in Figur 7 gezeigten, begleitenden Schallsignals mit spontanem Anstieg auf Maximalamplitude die Rissbildung definiert. Aus dem Abschwingverhalten des burstartigen Schallsignals kann auf die bei Rissbildung freigesetzte Schallenergie zurückgeschlossen werden. Hierbei gibt die zeitliche Amplitudenverteilung Aufschluss über die Primärenergie des Schallereignisses, unabhängig vom Laufweg der Schallquelle zum Schallaufnehmer. Diese Signale können mit Hilfe der Funktion I = Io D⁻ⁿ charakterisiert werden. Dabei ist I die gemessene Impulssumme eines Schallereignisses oberhalb einer Diskriminatorschwelle D und n ein Maß für das Abklingverhalten des Primärsignals, unabhängig vom Laufweg und der Dispersion, die I₀ beeinflussen.

### Bezugszeichenliste

- 1: Bein
- 2: Schallaufnehmer
- 3: Vorverstärker
- 4: Bandpassfilter
- 5: Zwischenverstärker

- 6: Messwerterfassung
- 7: Display
- 8: Gehäuse
- 9: Rand
- 10: Sonde

- 11: Druckfeder
- 12: Zwischenschicht

## Patentansprüche

1. Trainingsgerät mit einer Vorrichtung zur Aufnahme und Auswertung von während der Belastung in menschlichen Gelenken und/oder Knochen auftretenden Geräuschen, wobei besagte Vorrichtung einen ungedämpften, resonanten, piezoelektrischen Wandler als Schallaufnehmer (2) hat, der auf der Hautoberfläche eines Menschen aufsetzbar ist, und die eine Auswertelektronik aufweist, welche die von dem Schallaufnehmer (2) während der Belastung aufgenommenen Geräusche hinsichtlich des Auftretens von Mikrorissen im Knochen analysiert und daraus ein Warnsignal ableitet, falls die Belastung so hoch ist, dass eine Schädigung von Gelenken und/oder Knochen zu befürchten ist.

2. Trainingsgerät nach Anspruch 1, **dadurch gekennzeichnet, dass** die Auswertelektronik zusätzlich zur Auswertung der auf eine Rissbildung hinweisenden Geräusche zum Analysieren von auf eine Knorpelschädigung hinweisenden Geräuschen ausgebildet und das Trainingsgerät zur Anzeige dieser Geräusche ausgebildet ist.

3. Trainingsgerät nach Anspruch 2, **dadurch gekennzeichnet, dass** der Schallaufnehmer zum Befestigen mittels eines flüssigen, schallleitenden Klebers auf der Hautoberfläche ausgebildet ist.

4. Trainingsgerät nach zumindest einem der vorangehenden Ansprüche, **dadurch gekennzeichnet, dass** der Schallaufnehmer innerhalb eines hutförmigen Gehäuses (8) eine Sonde (10) mit einem Piezoelement hat, dass die Sonde (10) durch eine elastische, schalldämpfende Zwischenschicht (12) mit dem Gehäuse verbunden ist und dass zwischen der inneren Rückseite des Gehäuses und der Sonde eine die Sonde zur offenen Stirnseite des hutförmigen Gehäuses vorspannende Druckfeder angeordnet ist.

5. Trainingsgerät nach zumindest einem der vorangehenden Ansprüche, **dadurch gekennzeichnet, dass** seine Lager schallabsorbierend in einem Stützring aus austenitischem Werkstoff oder aus einem Mehrschichtverbundwerkstoff eingebaut sind.

6. Trainingsgerät nach zumindest einem der vorangehenden Ansprüche, **dadurch gekennzeichnet, dass** es Liege- oder Sitzflächen mit einer Auflage aus ungepolstertem, gummiertem Verbundwerkstoff aufweist.

## Claims

1. Training device with apparatus for receiving and evaluating sounds occurring in human joints and/or bones during loading, wherein said apparatus has an undamped, resonant piezoelectric transducer as sound receiver (2) which can be placed on the surface of the skin of a human being, and evaluation electronics which analyse the sounds picked up by the sound receiver (2) during loading for the occurrence of microcracks in the bone and derive a warning signal therefrom if the loading is so high as to pose a risk of injury to joints and/or bones.

2. Training device according to Claim 1, **characterized in that** the evaluation electronics are configured not only to evaluate sounds indicative of cracking but also to analyse sounds indicative of cartilage injury, and the training device is configured to indicate such sounds.

3. Training device according to Claim 1, **characterized in that** the sound receiver is designed to be attached to the surface of the skin by means of a liquid, sound-conducting adhesive.

4. Training device according to at least one of the preceding claims, **characterized in that** the sound receiver has a probe (10) with a piezoelectric element inside a hat-shaped casing (8), **in that** the probe (10) is connected to the casing by an elastic, sound-damping intermediate layer (12) and **in that** a compression spring urging the probe towards the open end of the hat-shaped casing is arranged between the inner rear end of the casing and the probe.

5. Training device according to at least one of the preceding claims, **characterized in that** its mounting is installed in a sound-absorbing manner in a support ring of austenitic material or of a multilayer composite material.

6. Training device according to at least one of the preceding claims, **characterized in that** it has resting or seating faces with a lining of unpadded, rubberized composite material.

## Revendications

1. Appareil d'exercice physique comprenant un dispositif pour détecter et interpréter des bruits qui se produisent pendant la sollicitation dans des articulations et/ou des os de personnes humaines, ledit dispositif comprenant, comme détecteur de son (2), un convertisseur piézoélectrique, non amorti, résonant, qui peut être placé sur une surface de la peau d'une personne et qui présente une électronique d'interprétation qui analyse les bruits détectés par le détecteur de son pendant la sollicitation pour détecter des microfissures dans l'os et qui en dérive un signal d'alarme dans le cas où la charge est si forte qu'on peut craindre une détérioration des articulations et/ou des os.

2. Appareil d'exercice physique selon la revendication 1, **caractérisé en ce que** l'électronique d'interprétation est construite pour effectuer en supplément de l'interprétation des bruits indicatifs d'une fissuration, l'analyse de bruits indicatifs une détérioration des cartilages et l'appareil d'exercice physique est construit pour afficher ces bruits.

3. Appareil d'exercice physique selon la revendication 2, **caractérisé en ce que** le capteur de son est constitué pour être fixé sur la surface de la peau au moyen d'une colle liquide qui conduit les sons.

4. Appareil d'exercice physique selon au moins une des revendications précédentes, **caractérisé en ce que** le capteur de son possède, à l'intérieur d'un boîtier (8) en forme de chapeau, une sonde (10) comportant un élément piézoélectrique, **en ce que** la sonde (10) est reliée au boîtier par une couche intermédiaire (12) élastique, isolante acoustique, et **en ce qu'**entre la face arrière intérieure du boîtier et la sonde, est disposé un ressort de compression qui précontraint la sonde vers le côté frontal ouvert du boîtier en forme de chapeau.

5. Appareil d'exercice physique selon au moins une des revendications précédentes, **caractérisé en ce que** ses portées sont montées dans une bague de support en matière austénitique ou en matière composite multicouche, dans un mode qui absorbe les sons.

6. Appareil d'exercice physique selon au moins une des revendications précédentes, **caractérisé en ce qu'**il présente des surfaces de couchage ou d'assise possédant un revêtement fait d'une matière composite caoutchoutée, non capitonnée.
